# EUROPÄISCHE PATENTANMELDUNG

(11) **EP 0 604 844 A1**
(43) Veröffentlichungstag der Anmeldung: **06.07.1994**
(21) Anmeldenummer: 93120271.7
(22) Anmeldetag: 16.12.1993
(51) Int. Cl.: C07C 219/08, C07C 233/36

(54) **Verfahren zur Herstellung von Trialklammoniumalkyl(meth)acryl-Verbindungen**

(30) Priorität: 22.12.1992 DE 4243466
(71) Anmelder: RÖHM GMBH, D-64293 Darmstadt (DE)
(72) Erfinder: Dr. Bartholomae Ines, D-64390 Erzhausen (DE); Dr. Hartwig, Drögemüller, D-64291 Darmstadt (DE); Kaul, Thomas, D-68642 Bürstadt (DE)

(57) **Zusammenfassung**

Die Erfindung betrifft ein Verfahren zur Herstellung von Trialkylammoniumalkyl(meth)acryl-Verbindungen der Formel I 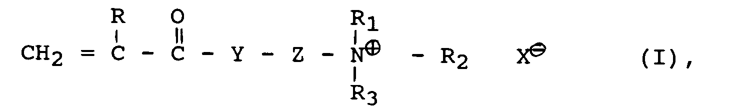
wobei
R für Wasserstoff oder Methyl; R₁, R₂, R₃ unabhängig voneinander für eine Alkylgruppe mit 1 bis 6 Kohlenstoffatomen; Y für Sauerstoff oder NR₄, wobei R₄ für Wasserstoff oder einen Alkylrest mit 1 bis 6 Kohlenstoffatomen steht; Z für eine gegebenenfalls verzweigte Alkylengruppe mit 1 bis 12 Kohlenstoffatomen; sowie X^{⊖} für das Anion aus dem alkylierenden Reagenz R₃X
stehen,
durch Umsetzung von den entsprechenden Dialkylaminoalkyl(meth)acryl-Verbindungen mit Alkylierungsmitteln R₃X in einem C1 bis C6-Alkohol als Reaktionsmedium im Temperaturbereich zwischen 0 und 100 Grad C, mit der Maßgabe, daß die Gesamtmenge an Dialkylaminoalkyl(meth)acryl-Verbindung und gegebenenfalls eine Teilmenge des Reaktionsmediums in einem Reaktionsgefäß vorgelegt werden und daß die Restmenge an Reaktionsmedium zusammen mit dem Quaternierungsmittel R₃X über die gesamte Reaktionsphase von maximal 3 Stunden dem Reaktionsansatz zudosiert wird.

## Beschreibung

### Gebiet der Erfindung

Die Erfindung betrifft ein Verfahren zur Herstellung von Trialkylammoniumalkyl(meth)acryl-Verbindungen durch Umsetzung von entsprechenden Dialkylaminoalkyl(meth)acryl-Verbindungen mit geeigneten Alkylierungsmitteln R₃X in niederen Alkoholen als Reaktionsmedium, wobei die Gesamtmenge der zu quaternierenden Dialkylaminoalkyl(meth)acrylverbindung, gegebenenfalls mit einem Teil des Reaktionsmediums, im Reaktionsgefäß vorgelegt wird und die Restmenge des Reaktionsmediums zusammen mit dem Alkylierungsmittel über die gesamte Reaktionsphase dem Reaktionsansatz kontinuierlich oder portionsweise zudosiert wird. Die Reaktionsprodukte weisen einen äußerst geringen Anteil an Umesterungs- und/oder Hydrolyseprodukten auf.

### Stand der Technik

Die Anwesenheit von Wasser bei der Quaternierung von tertiären Aminen, insbesondere von tertiären Aminoalkylestern oder tertiären Aminoalkylamiden der Acryl- oder der Methacrylsäure, führt häufig zur teilweisen Hydrolyse der Ester- oder der Amidgruppen, sowie in einigen Fällen zur Hydrolyse des Alkylierungsmittels. Damit werden die Quaternierungsprodukte von tertiären Aminoalkylestern der tertiären Aminoalkylamide der (Meth)acrylsäure mit dem unerwünschten Nebenprodukt (Meth)acrylsäure verunreinigt. Im gleichen Maße gilt dies für Polymerisate, die o.g. Quaternierungsprodukte enthalten.
Gemäß DE-PS 28 48 627 (= US-PS 4 239 876) erhält man unmittelbar polymerisationsfähige Mischungen aus Acrylamid und Quaternierungsprodukten von tertiären Aminoalkylestern oder tertiären Aminoalkylamiden der (Meth)acrylsäure in Abwesenheit von Wasser ohne Verwendung von weiteren Lösungsmitteln.
In der US-PS 4 096 133 wird die Quaternierung von Polymeren aus wasserlöslichen ethylenisch ungesättigten Aminen im sauren wäßrigen Medium durch Zugabe von Quaternierungsmittel, vorzugsweise Methylchlorid, und Methanol als Katalysator für die Quaternierung beschrieben.
US-PS 4 024 328 umfaßt die Umsetzung von Mannich-Basen, hervorgehend aus Polyacrylamid mit Alkylierungsmitteln in Gegenwart von katalytischen Mengen Methanol zu quaternierten Polydialkylaminoacrylamiden.
In einem Übersichtsartikel beschreibt J. Goerdeler die Quaternierung von Trimethylamin und Triethylamin in Methanol bzw. Ethanol (J. Goerdeler, Houben-Weyl 11/2, Seite 591ff, Georg Thieme Verlag, Stuttgart, 1958). Die US-PS 2 980 657 beschreibt die Herstellung von Polymerisaten enthaltend quaternierte Ammoniumgruppen durch Aminolyse von Polyacrylaten zu ternären Polyacrylamiden, die in absolutem Ethanol bei ca. 80 Grad C quaterniert werden.
In JP 82 120 560 (Chem. Abstr. 97, 198701y) wird die Quaternierung von monomeren Dialkylaminoalkyl(meth)acrylamiden mit Methylchlorid in Methanol bei Reaktionstemperaturen zwischen 10 und 50 Grad C beschrieben.

### Aufgabe und Lösung

Bei den Verfahren des Standes der Technik zur Quaternierung von monomeren Dialkylaminoalkyl(meth)acryl-Verbindungen, die mit Wasser in der Reaktionsphase arbeiten, entstehen Acryl- oder Methacrylsäure als unerwünschte Nebenprodukte durch Hydrolyse, deren Entfernung bei der Aufarbeitung der quaternären Ammoniumverbindungen äußerst aufwendig ist. Bei der Quaternierung polymerer Dialkylaminoalkyl(meth)acryl-Verbindungen sind solche Hydrolyse-Produkte in der Polymerkette fest eingebaut und können die Polymereigenschaften in unerwünschter Weise verändern, beispielsweise durch intermolekulare Assoziation via Wasserstoffbrücken, oder durch Salzbildung zwischen den Säure- und Aminogruppen, oder durch eine veränderte Hydrophilie der Polymeren.
Bei den im Stand der Technik referierten Verfahren zur Quaternierung von Dialkylaminoalkyl(meth)acryl-Verbindungen mit wasserfreiem Methanol bzw. Ethanol treten je nach Reaktionsbedingungen als Nebenprodukte durch Umesterung die entsprechenden Acryl- oder Methacrylsäureester auf. Die Abtrennung solcher (Meth)acrylsäureester aus dem Reaktionsgemisch ist ebenso wie die Abtrennung der (Meth)acrylsäure äußerst aufwendig.
Hieraus resultiert die Aufgabe, ein Verfahren zur Herstellung von guaternierten Dialkylaminoalkyl(meth)acryl-Verbindungen zur Verfügung zu stellen, das mit hohen Ausbeuten arbeitet, wobei die Quaternierungsprodukte keine oder nur sehr geringe Mengen an den o.g. Nebenprodukten als Verunreinigung aufweisen und wobei das Verfahren ohne aufwendige Trennschritte auskommt und hinterher eine gebrauchsfertige Lösung der quaternierten Dialkylaminoalkyl(meth)acrylate erhalten wird.
Überraschenderweise löst das im folgenden dargestellte erfindungsgemäße Verfahren diese Aufgabe ausgezeichnet. Es ist ein Verfahren zur Herstellung von Trialkylammoniumalkyl(meth)acryl-Verbindungen der Formel I: 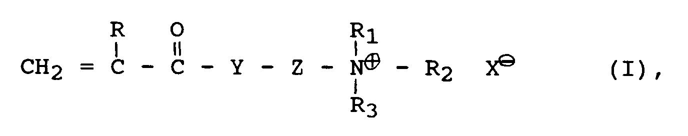
wobei:
R für Wasserstoff oder Methyl, R₁, R₂, R₃ unabhängig voneinander für eine Alkylgruppe mit 1 bis 6 Kohlenstoffatomen; Y für Sauerstoff oder NR₄, wobei R₄ für Wasserstoff oder einen Alkylrest mit 1 bis 6 Kohlenstoffatomen steht; Z für eine gegebenenfalls verzweigte Alkylengruppe mit 1 bis 12 C-Atomen; sowie X^{⊖}für das Anion aus dem quaternierenden Reagenz R₃X
stehen,
durch Umsetzung der entsprechenden Dialkylaminoalkyl(meth)acrylverbindungen mit Alkylierungsmitteln R₃X in einem C1- bis C6-Alkohol als Reaktionsmedium im Temperaturbereich zwischen 0 und 100 Grad C, gekennzeichnet dadurch, daß im Reaktionsgefäß die Gesamtmenge an Dialkylaminoalkyl(meth)acryl-Verbindung und gegebenenfalls eine Teilmenge des Reaktionsmediums vorgelegt wird und daß die Restmenge an Reaktionsmedium zusammen mit dem Alkylierungsmittel R₃X über die gesamte Reaktionsphase von maximal 3 Stunden dem Reaktionsansatz kontinuierlich oder portionsweise zudosiert wird.

In bevorzugten Ausführungsformen der Erfindung liegt der Anteil des Reaktionsmediums bei maximal 45 Gew.-% bezogen auf den Reaktionsansatz, die Reaktionstemperatur liegt zwischen 30 und 70 Grad C und der Druck im Reaktorgefäß ist über die gesamte Reaktionsphase konstant. Als quaternierte Reaktionsprodukte resultieren Trialkylammoniumalkyl(meth)acryl-Verbindungen von hoher Reinheit mit sehr geringen Anteilen an Nebenprodukten.

### Durchführung der Erfindung

Als Edukte für die Quaternierung werden Verbindungen der Formel II: 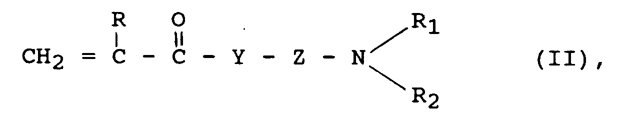
wobei
R für Wasserstoff oder Methyl; R₁ und R₂ unabhängig voneinander für eine Alkylgruppe mit 1 bis 6 Kohlenstoffatomen; Y für Sauerstoff oder NR₄, wobei R₄ für Wasserstoff oder einen Alkylrest mit 1 bis 6 Kohlenstoffatomen steht; sowie Z für eine gegebenenfalls verzweigte Alkylengruppe mit 1 bis 12 Kohlenstoffatomen
stehen,
und damit dieselbe Bedeutung wie die Substituenten in Formel I gemäß Anspruch 1 aufweisen,
eingesetzt.
Beispielhaft für Edukte gemäß Formel I seien genannt: 2-(N,N-Dimethylamino)ethyl(meth)acrylat, 4-(N,N-Dimethylamino)butyl(meth)acrylat, 2-(N-Ethyl-N-methylamino)ethyl(meth)acrylat, 2-Hydroxy-3-(N,N-Dimethylamino)propyl-(meth)acrylat, 2-(N,N-Diethylamino)ethyl(meth)acrylat, 3-(N,N-Dimethylamino)propyl(meth)acrylamid, 3-(N,N-Dimethylamino)-2,2-dimethylpropyl(meth)acrylamid oder 2-(N-Hydroxyethyl-N-methylamino)ethyl(meth)acrylamid.
Nach dem erfindungsgemäßen Verfahren wird die Gesamtmenge der zu quaternierenden Verbindung gemäß Formel II in einem Reaktionsgefäß, bevorzugt in einem Autoklaven, vorgelegt, wobei der Autoklav für Temperaturen von bis zu 200 Grad C und für Drücke von bis zu 50 bar ausgelegt ist.
In der Vorlage befindet sich neben der Verbindung gemäß Formel II ein Teil des Reaktionsmediums, vorzugsweise 0 bis 30 Gew.-%, besonders vorzugsweise 0 bis 10 Gew.-% bezogen auf den Reaktionsansatz. Der gesamte Anteil des Reaktionsmediums bezogen auf das Gemisch Reaktionsmedium/Verbindung gemäß Formel II beträgt maximal 50 Gew.-%, vorzugsweise maximal 45 Gew.-%. Als Reaktionsmedium verwendet werden bevorzugt wasserfreie C1-bis C6-Alkohole wie beispielsweise 1-Propanol, 2-Propanol, Ethanol, 1-Butanol, 2-Butanol, 3-Pentanol und besonders bevorzugt Methanol.
Während der Reaktionsphase, die maximal 3 Stunden, vorzugsweise maximal 2,5 Stunden dauert, wird das Quaternierungsmittel R₃X und der restliche Teil des Reaktionsmediums in separaten kontinuierlichen oder portionsweisen Massenströmen solchermaßen zudosiert, daß die Trialkylammoniumalkyl(meth)acryl-Verbindung gemäß Formel I 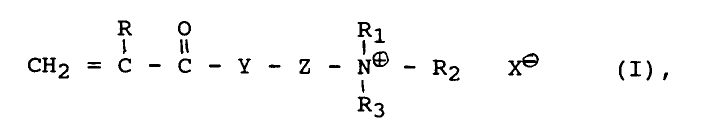
wobei
R für Wasserstoff oder Methyl, R₁, R₂, R₃ unabhängig voneinander für eine Alkylgruppe mit 1 bis 6 Kohlenstoffatomen, Y für Sauerstoff oder NR₄, wobei R₄ für Wasserstoff oder einen Alkylrest mit 1 bis 6 Kohlenstoffatomen steht, Z für eine gegebenenfalls verzweigte Alkylengruppe mit 1 bis 12 Kohlenstoffatomen, sowie X^{⊖}für das Anion aus dem quaternierenden Reagenz R₃X
stehen,
mit dem Reaktionsmedium eine rührfähige Suspension bildet.

Als Quaternierungsmittel R₃X können beispielsweise Alkylhalogenide wie Methylbromid, Methyliodid, Ethylchlorid oder Butyliodid oder vorzugsweise Methylchlorid, sowie weitere Alkylierungsmittel, wie Alkyltosylate oder Alkylsulfate wie Dimethylsulfat, eingesetzt werden. Im allgemeinen ist das Quaternierungsmittel R₃X im Reaktionsgemisch aus Verbindung gemäß Formel I und Reaktionsmedium gut löslich.
Das Quaternierungsmittel R₃X wird dem Reaktionsansatz im 1- bis 2-fachen, bevorzugt im 1,05 bis 1,50-fachen der stöchiometrischen Menge, die zur vollständigen Umsetzung der Verbindung gemäß Formel I notwendig ist, zugesetzt. Während der gesamten Reaktionsdauer beträgt die Reaktionstemperatur zwischen 0 und 100 Grad C, vorzugsweise zwischen 30 und 70 Grad C. Der Druck im Reaktionsgefäß ist über die gesamte Reaktionsdauer hinweg konstant und beträgt 10⁵ bis 6 x 10⁵ Pa, vorzugsweise 1,5 x 10⁵ bis 4 x 10⁵ Pa. Vorzugsweise wird der Druck durch die Dampfdrücke des Quaternierungsreagenz R₃X und des Reaktionsmediums aufrecht erhalten.

Nach Abschluß der Reaktion kann das überschüssige Quaternierungsreagenz R₃X und das Reaktionsmedium von der quaternierten Verbindung gemäß Formel I in üblicher Weise, beispielsweise durch Destillation oder durch Vakuumdestillation, abgetrennt werden. Ist das Quaternierungsreagenz R₃X bei den Reaktionstemperaturen gasförmig, so wird in einer bevorzugten Ausführungsform der Erfindung das überschüssige R₃X durch Einleiten von Luft in das Reaktionsgefäß aus dem Reaktionsansatz verdrängt.

Um die Polymerisation der Verbindungen gemäß Formel I und II während der Quaternierungsreaktion zu verhindern, sind während der gesamten Reaktionsdauer übliche Stabilisierungsmittel in Mengen von 0,01 bis 1 Gew.-%, vorzugsweise 0,01 bis 0,2 Gew.-% bezogen auf das Edukt der Formel II anwesend (vgl. beispielsweise auch Ullmann's Encyclopedia of Industrial Chemistry, Vol. A21, Seiten 158, 476, VCH Verlagsgesellschaft, Weinheim, 1992). Als Stabilisierungsmittel können beispielsweise verwendet werden: Hydrochinon, Triphenylphosphan, Triphenylphosphit, Bis-2,2,6,6-Tetramethyl-4-piperidylsebacat oder bevorzugt Hydrochinonmonomethylether. Vorzugsweise befindet sich das Stabilisierungsmittel gemeinsam mit der Verbindung der Formel II in der Vorlage bevor mit der Quaternierungsreaktion begonnen wird.

### Vorteile der Erfindung

Bei dem erfindungsgemäßen Verfahren zur Quaternierung von Dialkylaminoalkyl(meth)acryl-Verbindungen der Formel II fallen Trialkylammoniumalkyl(meth)acryl-Verbindungen von hoher Reinheit an. (Meth)acrylsäure als Nebenprodukt durch Hydrolyse der Verbindungen gemäß Formel II fällt durch das wasserfreie Reaktionsmedium nicht an. Auch die Alkoholyse der Verbindungen gemäß Formel II, die durch langes Einwirken des Reaktionsmediums und eine relativ große Konzentration des Reaktionsmediums bezogen auf den Gesamtansatz bei erhöhter Temperatur zu einer vermehrten Umsetzung der Verbindungen gemäß Formel II zu (Meth)acrylsäureestern führt, wird durch die spezielle Reaktionsführung auf ein Minimum reduziert.
Die Reaktionszeiten der erfindungsgemäßen Verfahren sind darüber hinaus deutlich geringer als diejenigen der Verfahren des Standes der Technik.

Die folgenden Beispiele sollen die Erfindung erläutern.

### BEISPIELE

### Beispiel 1

In einem Glasautoklaven mit Rührer, Innenthermometer und Manometer (Fa. SFS Zürich, 1 l Fassungsvermögen, max. Druck: 10 bar, max. Temperatur: 200 Grad C) werden 400,0 g 2-Dimethylaminoethylmethacrylat, 30,0 g Methanol und 0,6 g Hydrochinonmonomethylether vorgelegt. Nach dem Einstellen einer Reaktionstemperatur von 40 Grad C werden innerhalb von 1,5 Stunden 180,0 g gasförmiges Methylchlorid eingeleitet und 280,0 g Methanol über eine separate Zuleitung so zudosiert, daß die entstehende Suspension gerade noch rührfähig bleibt. Nach einer anschließenden Nachreaktionsphase von etwa 1 Stunde - Reaktorinnen- und Reaktormanteltemperatur haben den gleichen Wert - wird der Reaktor entspannt. Anschließend wird das überschüssige Methylchlorid aus dem flüssigen Reaktionsansatz innerhalb von 1,5 Stunden durch Lufteinleitung verdrängt. Eine gaschromatographische bzw. titrimetrische Analyse der methanolischen Lösung zeigt, daß bei einem vollständigen Umsatz von 2-Dimethylaminoethylmethacrylat 98,87 Gew.-% Reaktionsprodukt 2-Trimethylammoniumethylmethacrylatchlorid, 0,49 Gew.-% Methylmethacrylat und 0,69 Gew.-% Cholinchlorid (2-Hydroxyethyltrimethylammonium-chlorid) entstanden sind. Der Methanolgehalt der Lösung beträgt 35,12 Gew.-%. Die Zusammensetzung des gesamten Ansatzes ist: 35,12 Gew.-% Methanol, 0,32 Gew.-% Methylmethacrylat, 0,45 Gew.-% Cholinchlorid,
64,11 Gew.-% Trimethylammoniumethylmethacrylat-chlorid.

### Vergleichsbeispiel

In einem Glasautoklaven gemäß Beispiel 1 werden 400,0 g Dimethylaminoethylmethacrylat, 400,0 g Methanol und 0,28 g Hydrochinonmonomethylether vorgelegt. Nach dem Einstellen einer Reaktorinnentemperatur von 40 Grad C werden innerhalb von 2,2 Stunden 172,0 g gasförmiges Methylchlorid eingeleitet. Nach einer anschließenden Nachreaktionsphase von etwa 1 Stunde - Reaktorinnen- und Reaktormanteltemperatur haben den gleichen Wert - wird der Reaktor entspannt, wobei das Reaktionsprodukt 2-Trimethylammonium-ethylmethacrylat-chlorid in Lösung bleibt. Anschließend wird das überschüssige Methylchlorid aus dem flüssigen Reaktionsansatz innerhalb von 1,5 Stunden durch Lufteinleitung verdrängt.
Eine gaschromatographische bzw. titrimetrische Analyse der methanolischen Lösung zeigt, daß bei einem vollständigen Umsatz von 2-Dimethylaminoethylmethacrylat 63,83 Gew.-% 2-Trimethylammoniumethylmethacrylat-chlorid, 21,06 Gew.-% Cholinchlorid sowie 15,11 Gew.-% Methylmethacrylat entstanden sind. Die Zusammensetzung des gesamten Ansatzes ist: 42,87 % Methanol, 8,63 % Methylmethacrylat, 12,03 % Cholinchlorid, 36,47 % 2-Trimethylammoniumethylmethacrylat-chlorid.

## Patentansprüche

1. Verfahren zur Herstellung von Trialkylammoniumalkyl(meth)acryl-Verbindungen der Formel I 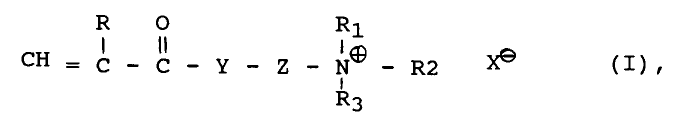 wobei
R für Wasserstoff oder Methyl; R₁, R₂, R₃ unabhängig voneinander für eine Alkylgruppe mit 1 bis 6 Kohlenstoffatomen; Y für Sauerstoff oder NR₄, wobei R₄ für Wasserstoff oder einen Alkylrest mit 1 bis 6 Kohlenstoffatomen steht; Z für eine gegebenenfalls verzweigte Alkylgruppe mit 1 bis 12 Kohlenstoffatomen sowie X^{⊖} für das Anion aus dem quaternierenden Reagenz R₃X
stehen,
durch Umsetzung von den entsprechenden Dialkylaminoalkyl(meth)acrylat-Verbindungen mit Alkylierungsmitteln R₃X in einem C1- bis C6-Alkohol als Reaktionsmedium im Temperaturbereich zwischen 0 und 100 Grad C.
dadurch gekennzeichnet,
daß die Gesamtmenge an Dialkylaminoalkyl(meth)acrylat-Verbindung und gegebenenfalls eine Teilmenge des Reaktionsmediums in einem Reaktionsgefäß vorgelegt
werden und daß die Restmenge an Reaktionsmedium zusammen mit dem Alkylierungsmittel R₃X über die gesamte Reaktionsphase von maximal 3 Stunden dem Reaktionsansatz zudosiert wird.

2. Verfahren zur Herstellung von Trialkylammoniumalkyl(meth)acryl-Verbindungen gemäß Anspruch 1, dadurch gekennzeichnet, daß der Gehalt an Reaktionsmedium bei maximal 45 Gew.-% bezogen auf den Reaktionsansatz liegt.

3. Verfahren zur Herstellung von Trialkylammoniumalkyl(meth)acryl-Verbindungen gemäß den Ansprüchen 1 und 2, dadurch gekennzeichnet, daß die Reaktionstemperatur zwischen 30 und 70 Grad C liegt.

4. Verfahren zur Herstellung von Trialkylammoniumalkyl(meth)acryl-Verbindungen gemäß den Ansprüchen 1 bis 3, dadurch gekennzeichnet, daß der Druck im Reaktionsgefäß während der gesamten Reaktionsphase konstant gehalten wird.

5. Verfahren zur Herstellung von Trialkylammoniumalkyl(meth)acryl-Verbindungen gemäß den Ansprüchen 1 bis 4, dadurch gekennzeichnet, daß X^{⊖} in der Formel I des Anspruchs 1 für Chlorid, Bromid, Iodid, Methylsulfat oder Tosylat steht.

6. Verfahren zur Herstellung von Trialkylammoniumalkyl(meth)acryl-Verbindungen gemäß den Ansprüchen 1 bis 5, dadurch gekennzeichnet, daß das Reaktionsmedium Methanol ist.

7. Verwendung der gemäß den Ansprüchen 1 bis 6 hergestellten Trialkylammoniumalkyl(meth)acryl-Verbindungen zur Herstellung von Polymerisaten .
